# EUROPEAN PATENT APPLICATION

(11) **EP 2 433 551 A1**
(43) Date of publication of application: **28.03.2012**
(21) Application number: 11180451.4
(22) Date of filing: 07.09.2011
(51) Int. Cl.: A61B 1/018, A61B 1/005, A61B 1/05, A61B 1/06, A61B 1/273, A61B 1/01

(54) **Endoscope apparatus**

(30) Priority: 28.09.2010 JP 2010217960
(71) Applicant: Fujifilm Corporation, Minato-ku Tokyo (JP)
(72) Inventor: IWASAKA, Masayuki, Kanagawa (JP); OHKI, Tomohiro, Kanagawa (JP)
(74) Representative: Klunker . Schmitt-Nilson . Hirsch

(57) **Abstract**

An endoscope apparatus (100) includes a forceps port (33) through which a treatment instrument (27) is inserted, an observation window (37) for observing an object and an illumination window (45) which are arranged on a distal-end surface (35) of an endoscope insertion portion (13) having a bendable part (17). An opening circumferential-edge (39) of the forceps port (33) is arranged to protrude from an axially distal-end of the endoscope insertion portion (13). The distal-end surface (35) between the opening circumferential-edge (39) and an edge of an outer circumferential surface (41) of the endoscope insertion portion (13) is a sloping surface to form an end of the endoscope having a taper shape so as to protrude the opening circumferential-edge (39). Preferably, the observation window (37) is arranged at a position such that a part of the opening circumferential-edge (39) of the forceps port (33) is within an observation view field (73) of the observation window (37).

## Description

### BACKGROUND OF THE INVENTION

### 1. Technical Field

The present invention relates to an endoscope apparatus.

### 2. Description of Related Art

Bile/pancreatic duct endoscopes for observing the inside of a bile duct or a pancreatic duct by being inserted thereinto are utilized for the diagnosis of the stenosis of a bile duct or a pancreatic duct, the distinction of the benignancy or malignancy of a protruding lesion, and the like. The bile/pancreatic duct endoscope (i.e., a baby endoscope) has a small-diameter insertion portion having a diameter of about 3 millimeters (mm). The bile/pancreatic duct endoscope is used by inserting the insertion portion into a forceps port of an ordinary endoscope (i.e., a mother endoscope). The baby endoscope is operated as follows. That is, after the endoscope distal end of the mother endoscope is inserted up to a duodenum, the baby endoscope is led out of the distal end of the insertion portion of the mother endoscope. Then, the insertion portion of the baby endoscope is inserted from a duodenal papilla into the bile duct or the pancreatic duct. The two endoscopes, i.e., the mother endoscope and the baby endoscope are operated in such manners, respectively, to thereby enable the observation of the inside of the bile duct or the pancreatic duct (see JP-A-2010-063772).

However, to simultaneously operate the two endoscopes, operators respectively operating the endoscopes are needed. Both the endoscopes are operated in cooperation with each other so that manipulations for operating the endoscopes become complex and that the operators need skills. Accordingly, there are demands for observing the inside of the bile duct or the pancreatic duct by simply introducing a single endoscope thereinto. However, to insert the insertion portion of the endoscope into the duodenal papilla, the endoscope needs thinness suitable for inserting the insertion portion of the endoscope thereinto. On the other hand, it is impossible for such a small-diameter bile/pancreatic duct endoscope to reach the duodenum by itself from an oral cavity.

In addition, in JP-A-2004-351138, an endoscope is described, which the distal-end portion thereof is protruded like a spatula to thereby enhance the ability to insert the endoscope into a narrowed body cavity tube. The endoscope is configured to be inserted thereinto while the tapered spatula-like distal-end portion of the endoscope is pressed against a living-body tissue.

### SUMMARY

However, the endoscope disclosed in JP-A-2004-351138 is still large in insertion resistance even if the distal-end portion thereof is formed like a spatula, when the distal-end portion thereof is inserted into a particularly narrow body cavity tube. In addition, an observation window is arranged forward of a forceps port, a distal-end part of a treatment instrument is difficult to come into sight. When the insertion portion of the endoscope is inserted into the bile duct or the pancreatic duct, it is necessary for cutting the duodenal papilla to improve the visibility of the treatment instrument protruded from the forceps port. In addition, it is also necessary to assure the observation view field without hiding the boundary between a cut part and an uncut part of the duodenal papilla with the treatment instrument.

An object of the invention is to provide an endoscope apparatus configured so that a distal-end of the insertion portion thereof can smoothly be inserted into small-diameter bile and pancreatic ducts, and that manipulations performed with a treatment instrument can easily be observed.

The invention is constituted as below.

An endoscope apparatus includes a forceps port, an observation window and an illumination window which are arranged on a distal-end surface of an endoscope insertion portion having a bendable bending portion. A treatment instrument is inserted through the forceps port. The observation window observes an object. An opening circumferential-edge of the forceps port is arranged to protrude from an axially distal-end of the endoscope insertion portion. The distal-end surface between the opening circumferential-edge and an edge of an outer circumferential surface of the endoscope insertion portion is a sloping surface to form an end of the endoscope having a taper shape so as to protrude the opening circumferential-edge.

An endoscope apparatus according to the invention, a distal-end of the insertion portion thereof can smoothly be inserted into small-diameter bile and pancreatic ducts. In addition, manipulations performed with a treatment instrument can easily be observed.

### BRIEF DESCRIPTION OF THE DRAWINGS

FIG. 1 is a diagram for illustrating an embodiment according to the invention, which is an external view of an endoscope apparatus according to the embodiment.
FIG. 2 is a perspective view illustrating a distal-end portion of the endoscope apparatus illustrated in FIG. 1.
FIG. 3 is a front view illustrating a distal-end surface in front view of the distal-end portion of the endoscope apparatus illustrated in FIG 2.
FIG. 4 is a cross-sectional view, taken on first straight line illustrated in FIG. 3, illustrating the distal-end portion of the endoscope apparatus.
FIG. 5 is an enlarged view of a bending portion of the endoscope apparatus illustrated in FIG. 1.
FIG. 6 includes a front view of a vicinity of a duodenum and a partially cutaway enlarged view of a primary part of the vicinity of the duodenum.
FIG. 7A is a side view of the distal-end portion of the endoscope that faces a duodenal papilla when a body cavity is cut, and FIG. 7B is a side view of the distal-.end portion of the endoscope apparatus inserted into a bile duct.
FIG. 8A is a perspective view of the distal-end portion of the endoscope apparatus, on which an observation window is arranged on a slope that is inclined at an angle of inclination and continuous to another slope formed around a circumferential edge of an opening at a different angle of inclination, and FIG. 8B is a front view of the distal-end portion illustrated in FIG. 8A.

### DETAILED DESCRIPTION OF EMBODIMENTS OF THE INVENTION

Hereinafter, an embodiment of the invention is described with referee to the accompanying-drawings.

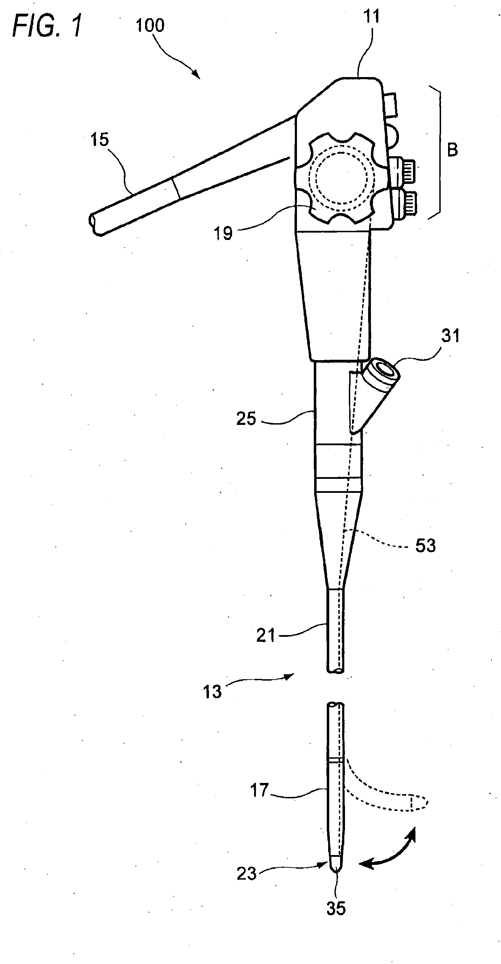

FIG. 1 is a diagram for illustrating the embodiment according to the invention. More particularly, FIG. 1 is an external view of an endoscope apparatus according to the embodiment.

An endoscope 100 includes an endoscope operation portion 11, and an endoscope insertion portion 13 that is provided continuously to the endoscope operation portion 11 and inserted into a body cavity. A universal cord 15 containing various duct lines and signal cables is connected to the endoscope operation portion 11. A connector to be detachably connected to a control apparatus (not shown) is attached to a distal-end portion of the universal cord 15. Various buttons B, such as an air-supply/water-supply button, a suction button, a shutter button, and a function switching button, are provided on the endoscope operation portion 11 to be juxtaposed with one another. In addition, an angle knob 19 for bending a bending portion 17 provided at the distal-end side of the endoscope insertion portion 13.

The endoscope insertion portion 13 is configured by a flexible portion 21, a bending portion 17, and a distal-end portion (endoscope distal-end portion) 23, which are arranged from the side of the endoscope operation portion 11 in this order. The flexible portion 21 has flexibility and is provided continuously to a base end side of the bending portion 17. The bending portion 17 is bent by turning the angle knob 19 of the endoscope operation portion 11 to pull a bending wire inserted into the endoscope insertion portion 13. Accordingly, the endoscope distal-end portion 23 can be directed to a desired direction.

A continuously provided portion 25 between the endoscope operation portion 11 and the endoscope insertion portion 13 is provided with a treatment instrument insertion portion 31 for inserting a treatment instrument into a treatment instrument insertion channel to which a treatment instrument, a water-supply means and the like are inserted. The treatment instrument insertion channel is formed to extend from the endoscope distal-end portion 23 to the endoscope operation portion 11 such that various treatment instruments such as a diathermy knife can be inserted thereinto.

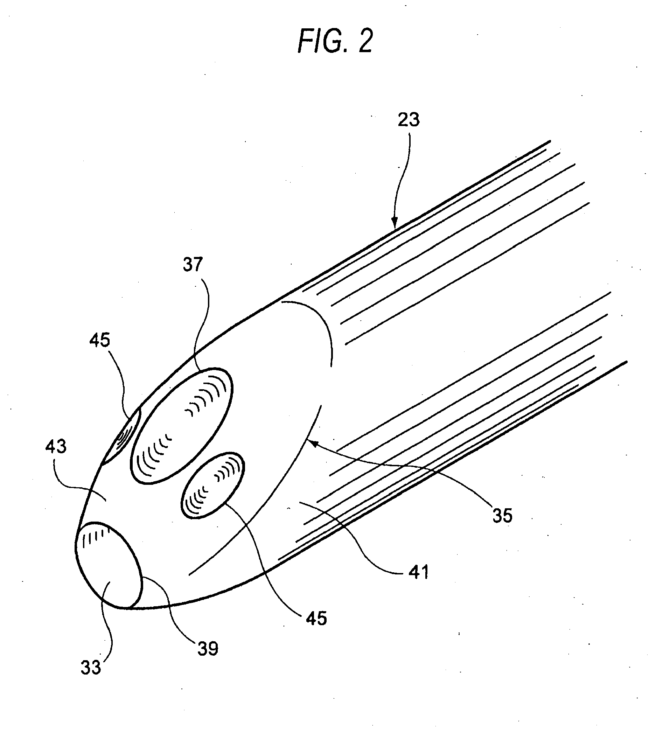

FIG. 2 is a perspective view of the endoscope distal-end portion 23 illustrated in FIG. 1.

The endoscope apparatus 100 is configured such that a forceps port 33 into which the treatment instrument is inserted, and an observation window 37 for observing an object are arranged on a distal-end surface 35 of the endoscope insertion portion 13 having the bendable bending portion 17. An opening circumferential-edge 39 of the forceps port 33 is arranged to protrude from an axially distal-end of the endoscope insertion portion 13. The distal-end surface 35 between the opening circumferential-edge 39 and an edge of an outer circumferential surface 41 of the endoscope insertion portion 13 is a sloping surface to form an end of the endoscope having a taper shape so as to protrude the opening circumferential-edge.

The observation window 37 and a pair of illumination windows 45 arranged opposite to each other across the observation window 37 are provided on the endoscope distal-end portion 23. Illumination light is output from the illumination windows 45. An observed image is acquired through the observation window 37. Light output from a light source portion of the above control apparatus is supplied to the endoscope apparatus 100 through the connector and the universal cord 15. The light is irradiated from the illumination window 45 of the endoscope distal-end portion 23 through a light guide.

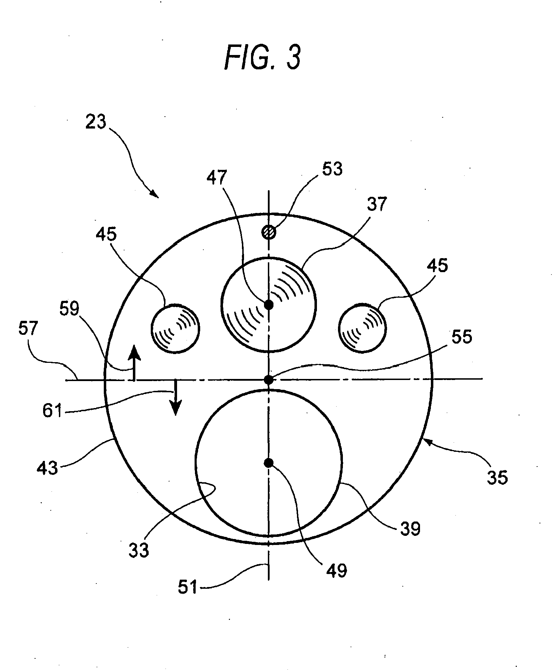

FIG. 3 is a front view of the distal-end surface of the endoscope distal-end portion 23 in front view illustrated in FIG. 2.

When the endoscope distal-end portion 23 is viewed from front, the pair of illumination windows 45 is arranged on a straight line passing through the center of each of the observation window 37 and the forceps port 33. The straight line passing through the center of each of the observation window 37 and the forceps port 33 is passed through the center of the endoscope distal-end portion 23. The forceps port 33, the observation windows 37, and the operating wire 53 are arranged on a first straight line 51 connecting an observation window center 47 and a forceps port center 49 on the distal-end surface of the endoscope distal-end portion 23. In addition, the operating wire 53 is arranged opposite to the forceps port 33 across the observation window 37. The observation window 37 is arranged in one of two regions into which the distal-end surface of the endoscope distal-end portion 35 is divided by a second straight line 57 that passes through the center 55 of the distal-end surface of the endoscope 35 in front view and that is perpendicular to the first straight line 51. The forceps port 33 is arranged in the other region 61. The operating wire 53 is arranged in the one of the above regions 59.

That is, the second straight line 57 is orthogonal to the first straight line 51 connecting the observation window center 47 and the forceps port center 49 on the endoscope distal-end portion 23 in axial front view. In addition, the second straight line 57 divides, into two regions, the distal-end surface of the endoscope distal-end portion 23 in axial front view. The observation window 37 and the operating wire 53 are arranged in one 59 of the regions, the boundary between which is the second straight line 57. The forceps port 33 is arranged in the other region 61.

With such an arrangement, the waste of the installation space of the observation window 37 is eliminated. Then, the saved space can be appropriated for the forceps port 33. Consequently, a large space can be assured as the installation space of the forceps port 33.

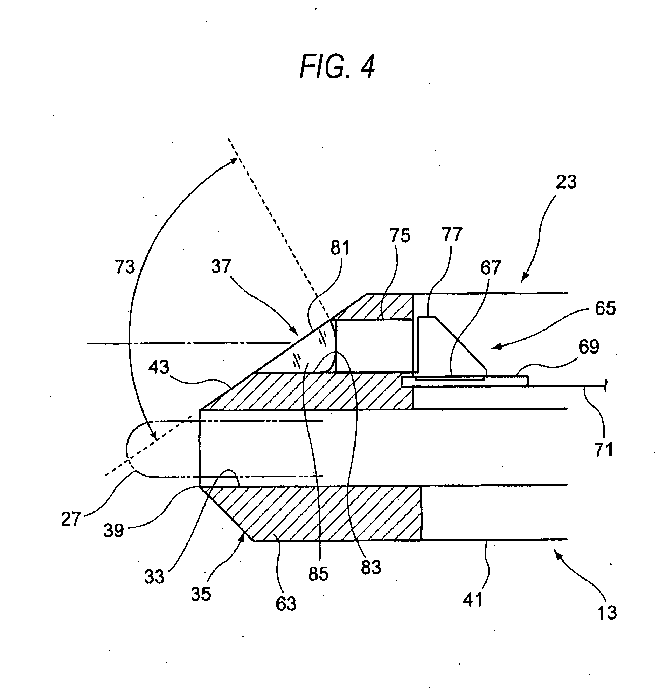

FIG. 4 is a cross-sectional view taken on the first line 51 illustrated in FIG. 3.

The endoscope distal-end portion 23 has a circular-cylindrically-shaped distal-end rigid portion 63 made of e.g., ceramics, and stainless steel. An imaging optical system 65 is provided in the distal-end rigid portion 63. The imaging optical system 65 includes an imaging device 67 for imaging an observed region illuminated with an illumination optical system, and a substrate 69 on which the imaging device 67 is mounted. An imaging signal representing an observed image obtained from the imaging device 67 is output to the control apparatus through a signal line 71 connected to the substrate 69. The control apparatus causes a monitor (not shown) to display image information obtained by performing image processing on the imaging signal input thereto. Instructions representing such a sequence of processing can be input from an input portion, such as a keyboard or a mouse, connected to the control apparatus. A charge-coupled device (CCD) image sensor and a complementary metal-oxide semiconductor (CMOS) image sensor are used as the imaging device 67 of the imaging optical system 65.

The imaging optical system 65 provided inside the observation window 37 is such that an image is formed on the imaging device 67 mounted on the substrate 69 from light taken in from an objective lens group configured by a plurality of objective lenses housed in a barrel 75 by turning the optical path of the light with a triangular prism 77 a right angle. Image signals based on the image information taken in by the imaging device 67 is output through the signal line 71 connected to the substrate 69. With this configuration, the imaging device 67 can be arranged in parallel with the central axis of the endoscope distal-end portion 23, using the triangular prism 77. Thus, the imaging device 67 and a lens unit can be housed efficiently in space and compactly.

The observation window 37 is arranged at a position at which a part of the opening circumferential-edge 39 of the forceps port 33 falls within an observation view field 73 from the observation window 37. Thus, the forceps port 33 is arranged at the most distal-end in an axial direction. Consequently, the treatment instrument 27 protruded from the forceps port 33 can be made to fall within the observation view field 73 at an early stage. Accordingly, a treatment with the endoscope can smoothly be performed.

In addition, the observation window 37 includes an inclined outer-surface 81 extending along the distal-end surface of the endoscope distal-end portion 35. A cover lens 85 is provided at a distal-end portion of an observation through hole 83. An outer surface 81 of the cover lens 85 is the same as the inclined surface 43. With such a configuration, the observation window 37 is a part of the inclined surface 43. The insertability of the endoscope is enhanced.

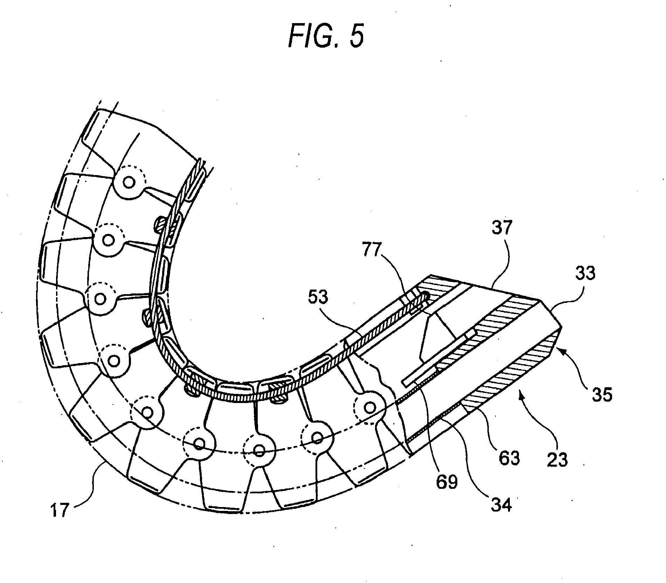

FIG. 5 is an enlarged cross-sectional view of the bending portion 17 of the endoscope apparatus 100 illustrated in FIG. 1.

The bending portion 17 is bent by pulling the above operating wire 53 such that a side thereof, in which the operating wire 53 is arranged, is set as an inner side of the bend of the bending portion 17. Accordingly, the operating wire 53 is arranged opposite to the forceps port 33 connected to a forceps channel 34 across the observation window 37. The operating wire 53, the observation window 37, and the forceps port 33 are serially arranged from the inner side in a bending direction toward an outer side of the bend thereof.

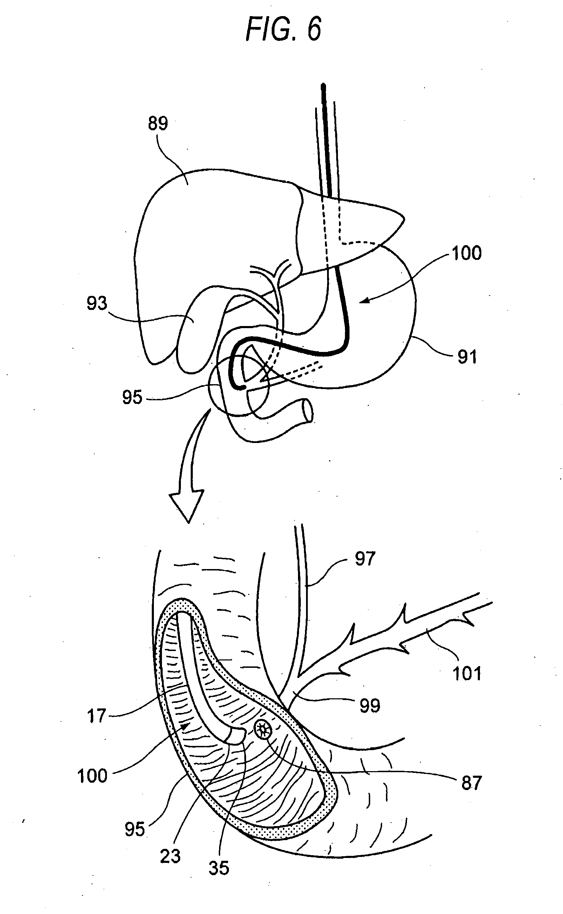

With such an arrangement/configuration, the treatment instrument 27 protruded from the forceps port 33 can visually be recognized from the inner side of the bend. For example, when a duodenal papilla 87 (see FIG. 6) to be located at the inner side of the bend is cut, the view field is assured without hiding the boundary between a cut part and an uncut part of the duodenal papilla with the treatment instrument 27. In a state illustrated in FIG. 5, the duodenal papilla 87 is located in front of or below the observation window 37. In a case where cutting proceeds towards an upper part illustrated in FIG. 5, if the positions of the observation window 37 and the forceps port 33 are inverted, the boundary between the cut part and the uncut part is hidden by the treatment instrument 27. According to the present configuration, the manipulation by the treatment instrument 27 can surely be observed, without occurrence of such a situation.

After the endoscope apparatus 100 is bent by pulling the operating wire 53, if the bent-state is canceled, and if the operating wire 53 is loosened by operating the angle knob 19, an elastically restoring force of a resin forceps tube configuring the forceps channel 34 of the forceps port 33 facilitates the restoration of the linear shape of the endoscope apparatus 100.

Next, endoscopy of a bile duct is described hereinafter as an example of an operation of the endoscope apparatus 100 having the above configuration.

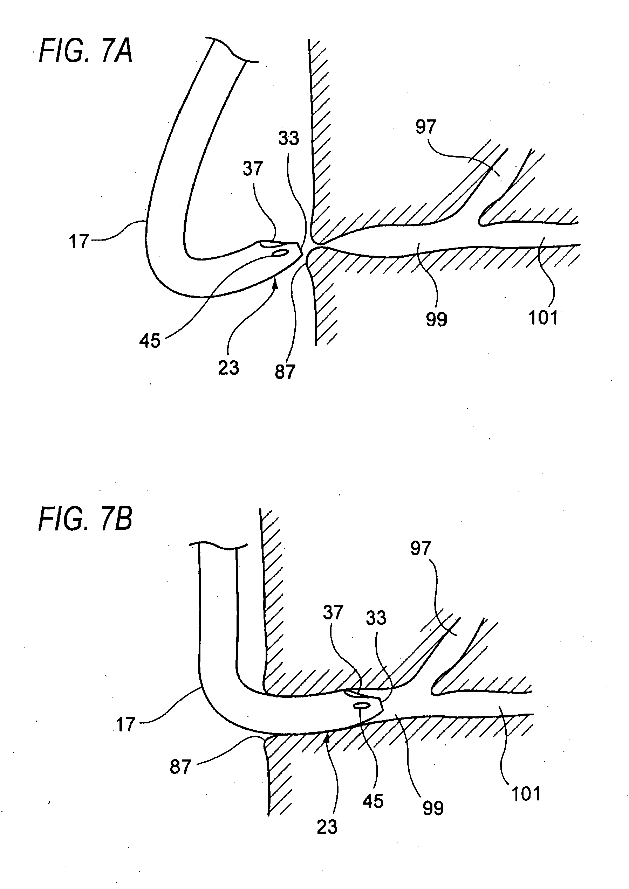

FIG. 6 includes a front view of a liver 89, a stomach 91, and a cholecyst 93 in the vicinity of a duodenum 95, and a partially cutaway enlarged view of a primary part of each of the liver 89, the stomach 91, and the cholecyst 93 in the vicinity of the duodenum 95. The distal-end portion 23 of the endoscope apparatus 100 is inserted into the position of the duodenum 95 by an operator. A common duct 99 to which the bile duct 97 and the pancreatic duct 101 are connected is connected to the duodenum 95. At the connection region between the common duct 99 and the duodenum 95, a duodenal papilla 87 is opened. The operator operates the angle knob 19 to inflect the bending portion 17. As illustrated in FIG. 7A, the endoscope distal-end portion 35 is arranged to face the duodenal papilla 87.

Then, the operator causes the treatment instrument, which is a diathermy knife, to protrude from the forceps port 33 (see FIG. 2). While viewing an imaged image obtained through the observation window 37, the operator cuts the duodenal papilla 87 with the diathermy knife. Upon completion of cutting the duodenal papilla 87, the operator inserts the endoscope distal-end portion 23 into the cut duodenal papilla, as illustrated in FIG. 7B. Then, the operator inserts the endoscope distal-end portion 23 into the bile duct 97 from the common duct 99. Thus, the endoscopic observation of the inside of the bile duct is performed.

When the inside of the bile duct 97 is observed with the endoscope distal-end portion 23 inserted into the common duct 99 and the bile duct 97, preferably, a transparent fluid such as a physiologic saline solution is supplied and discharged thereto and therefrom in order to make up for visual field deterioration due to yellowish bile. At that time, because the forceps port 33 is arranged at the most distal-end, the supply and discharge of the transparent fluid can instantaneously be performed. In addition, the view field from the observation window can be assured. The same holds in the case of inserting the endoscope apparatus into a pancreatic duct 101.

In the endoscope apparatus 100, the distal-end portion of the endoscope insertion portion 13 is sharpened. Thus, the insertion of the endoscope apparatus into a particularly narrow body cavity can smoothly be performed. In addition, the range of utilization of the endoscope apparatus 100 can be enlarged. On the other hand, the forceps port 33 is arranged at the protruded distal-end portion. Thus, even when the endoscope distal-end portion 23 is not completely inserted into a body cavity duct, a body fluid or waste-material contained in a body cavity can be sucked. Thus, treatments can simply be performed. In addition, the securement of the view field is facilitated.

Next, modifications of the tapered slope of the endoscope distal-end portion are described hereinafter.

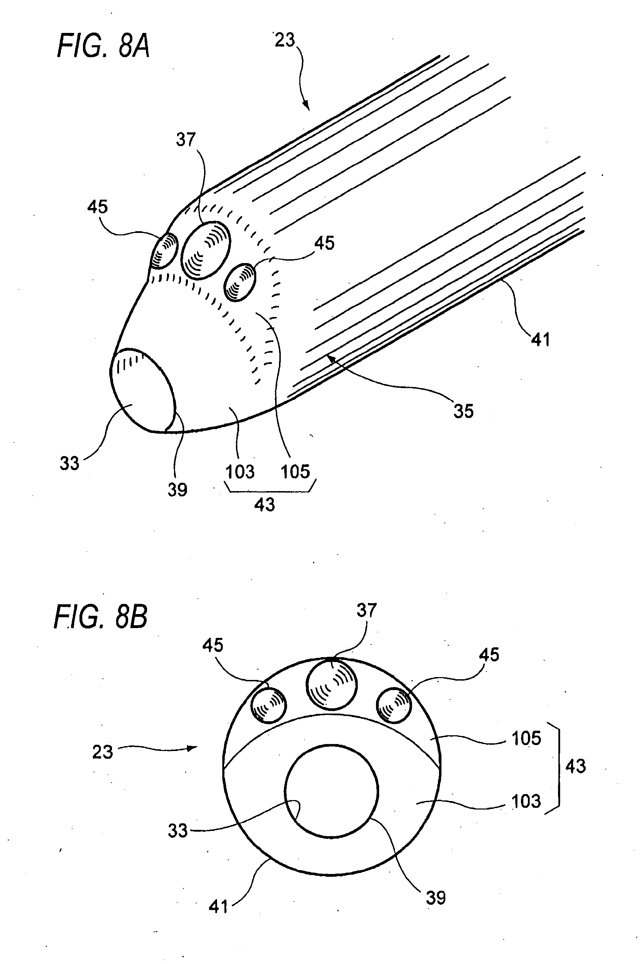

FIG. 8A is a perspective view of the endoscope distal-end portion 23, on which the observation window 37 is arranged on a slope that is inclined at an angle of inclination and continuous to another slope 43 formed around the circumferential edge 39 of an opening at a different angle of inclination. FIG. 8B is a front view of the distal-end portion illustrated in FIG. 8A.

In the foregoing description, an example of the endoscope apparatus 100 has been described, in which the distal-end surface extending from the opening circumferential-edge 39 formed on the endoscope distal-end portion 35 to a part, to which the outer circumferential-surface 41 of the endoscope insertion portion 13 is connected, is formed on the tapered slope 43 from which the opening circumferential-edge 39 is protruded. However, the slope 43 can be configured by a plurality of slopes, i.e., the first slope 103 and the second slope 105.

In this case, the first slope 103 is continuous to the opening circumferential-edge 39 of the forceps port 33. The second slope 105 is erected at an erection angle with respect to the central axis of the endoscope distal-end portion 23, which is larger than an angle at which the first slope 103 is erected. The erection angle is set within a range of angles, in which the insertion resistance is not increased. With such a configuration, while good insertability is assured due to the first slope 103, incidence of observation light is facilitated by erecting a light incidence surface of the observation window 37 on the second slope 105.

Accordingly, according to the above endoscope apparatus 100, the distal-end portion of the endoscope insertion portion 13 can smoothly be inserted into small-diameter bile and pancreatic ducts. In addition, the manipulation using the endoscope can be observed more clearly.

Thus, the invention is not limited to the above embodiments and can be modified and applied by those skilled in the art, based on the description of the present specification and known techniques. Such modification and application are intended by the invention and included in the scope of the invention for protection.

As described below, the following matter is disclosed in this specification.
(1) An endoscope apparatus includes a forceps port, an observation window and an illumination window which are arranged on a distal-end surface of an endoscope insertion portion having a bendable bending portion. A treatment instrument is inserted through the forceps port. The observation window observes an object. An opening circumferential-edge of the forceps port is arranged to protrude from an axially distal-end of the endoscope insertion portion. The distal-end surface between the opening circumferential-edge and an edge of an outer circumferential surface of the endoscope insertion portion is a sloping surface to form an end of the endoscope having a taper shape so as to protrude the opening circumferential-edge.

According to this endoscope apparatus, the distal-end portion of the endoscope insertion portion is sharpened. Thus, the insertion of the endoscope apparatus into a particularly narrow body cavity can smoothly be performed. In addition, the range of utilization of the endoscope apparatus can be enlarged. On the other hand, the forceps port is arranged at the protruded distal-end portion. Thus, even when the endoscope distal-end portion is not completely inserted into a body cavity duct, a body fluid or waste-material contained in a body cavity can be sucked. In addition, the securement of the view field is facilitated.
(2) The endoscope apparatus according to (1), the observation window is arranged at a position such that a part of the opening circumferential-edge of the forceps port is within an observation view field of the observation window.

According to this endoscope apparatus, the forceps port is arranged at the most axially distal-end. Consequently, the treatment instrument protruded from the forceps port can be made to fall within the observation view field at an early stage. Accordingly, a treatment with the endoscope can smoothly be performed.
(3) The endoscope apparatus according to (1) or (2), the observation window has an inclined outer surface extending along the distal-end surface.

According to this endoscope apparatus, the observation window is a part of the slope. Thus, the endoscope apparatus can smoothly be inserted.
(4) The endoscope apparatus according to any one of (1) to (3), the forceps port, the observation window, and an operating wire are arranged on a first straight line connecting a center of the observation window and a center of the forceps port. The operating wire is arranged opposite to the forceps port across the observation window.

According to this endoscope apparatus, the treatment instrument protruded from the forceps port can visually be recognized from the inner side of the bend. For example, in a case where a duodenal papilla to be located on the inner side of the bend is cut, the view field can be assured without hiding the boundary between the cut part and the uncut part with the treatment instrument. In addition, the manipulation performed treatment instrument can easily be observed.
(5) The endoscope apparatus according to (4), a second straight line passes through a center of the distal-end surface in a front view thereof and is orthogonal to the first straight line. The observation window is arranged on one of two regions into which the distal-end surface is divided by the second straight. The forceps port is arranged on the other region.

According to this endoscope apparatus, the waste of the installation space of the observation window can be eliminated. Then, the saved space can be appropriated for the installation space of the forceps port. Consequently, a large space can be assured as the installation space of the forceps port. Incidentally, at the inner side of the observation window, the imaging device and the lens unit can be housed efficiently in space and compactly.

## Claims

1. An endoscope apparatus comprising: a forceps port through which a treatment instrument is inserted; an observation window for observing an object; and an illumination window which are arranged on a distal-end surface of an endoscope insertion portion having a bendable bending portion,
wherein an opening circumferential-edge of the forceps port is arranged to protrude from an axially distal-end of the endoscope insertion portion, and
the distal-end surface between the opening circumferential-edge and an edge of an outer circumferential surface of the endoscope insertion portion is a sloping surface to form an end of the endoscope having a taper shape so as to protrude the opening circumferential-edge.

2. The endoscope apparatus according to claim 1,
wherein the observation window is arranged at a position such that a part of the opening circumferential-edge of the forceps port is within an observation view field of the observation window.

3. The endoscope apparatus according to claim 1 or 2,
wherein the observation window has an inclined outer surface extending along the distal-end surface.

4. The endoscope apparatus according to any one of claims 1 to 3,
wherein the forceps port, the observation window, and an operating wire are arranged on a first straight line connecting a center of the observation window and a center of the forceps port, and
the operating wire is arranged opposite to the forceps port across the observation window.

5. The endoscope apparatus according to claim 4,
wherein a second straight line passes through a center of the distal-end surface in a front view thereof and is orthogonal to the first straight line,
the observation window is arranged on one of two regions into which the distal-end surface is divided by the second straight, and
the forceps port is arranged on the other region.
